# EUROPÄISCHE PATENTANMELDUNG

(11) **EP 1 184 010 A2**
(43) Veröffentlichungstag der Anmeldung: **06.03.2002**
(21) Anmeldenummer: 01120562.2
(22) Anmeldetag: 29.08.2001
(51) Int. Cl.: A61F 9/007

(54) **Kammerwasser-Drainagevorrichtung**

(30) Priorität: 29.08.2000 DE 10042310
(71) Anmelder: Aixmed Gesellschaft für Medizintechnik mbH, 52222 Stolberg (DE)
(72) Erfinder: Jansen, Matthias, 52223 Stolberg (DE); Keune, Dirk, 52223 Stolberg (DE); Roosenboom, Bernd, 52134 Herzogenrath (DE)
(74) Vertreter: Bauer, Dirk, Dipl.-Ing. Dipl.-Kfm.

(57) **Zusammenfassung**

Eine Kammerwasser-Drainagevorrichtung (10) für ein Auge (2) weist eine die Sklera (11) durchdringende und ein inneres Lumen (18) begrenzende Drainageleitung (13) auf, die so plazierbar ist, daß ihr distales Ende (19) in der vorderen oder hinteren Kammer (1) des Auges (2) angeordnet ist. Außerdem weist die Vorrichtung (10) einen an das proximale Ende der Drainageleitung (13) angeschlossenen Verteilkörper auf, der außerhalb der vorderen oder hinteren Kammer (1) angeordnet ist und eine Verteilung des abgeleiteten Kammerwassers an ihr umgebendes Gewebe bewirkt. Der Verteilkörper ist eine Halteplatte (12), die fest mit der Drainageleitung (13) verbunden und ungefähr senkrecht zu deren proximalen Endabschnitt ausgerichtet ist, radial über deren Außendurchmesser vorsteht und eine Durchtrittsöffnung (20) aufweist, die koaxial zu der Drainageleitung (13) verläuft und eine Querschnittsfläche besitzt, die mindestens der minimalen Querschnittsfläche des Lumens (18) der Drainageleitung (13) entspricht. Um eine leicht implantierbare und ohne weiteres fixierbare Vorrichtung (10) zu erhalten, wird vorgeschlagen, daß daß die Drainageleitung (13) mit einem Druckentlastungsventil (26, 29) versehen ist, das einen Kammerwasserabfluß erst oberhalb eines bestimmten Drucks erlaubt, während der Implantation der Vorrichtung (1) von einer Nadel zerstörungsfrei durchdringbar und dabei reversibel in seinem Öffnungsquerschnitt im wesentlichen auf den Querschnitt der Drainageleitung aufweitbar ist.

## Beschreibung

Die Erfindung betrifft eine Kammerwasser-Drainagevorrichtung für ein Auge mit einer die Sklera durchdringenden und ein inneres Lumen begrenzenden Drainageleitung, die so plazierbar ist, daß ihr distales Ende in der vorderen oder hinteren Kammer des Auges angeordnet ist, und einem an das proximale Ende der Drainageleitung angeschlossenen Verteilkörper, der außerhalb der vorderen oder hinteren Kammer angeordnet ist und eine Verteilung des abgeleiteten Kammerwassers an ihn umgebendes Gewebe bewirkt, wobei der Verteilkörper eine Halteplatte ist, die fest mit der Drainageleitung verbunden ist.

Eine derartige Vorrichtung ist beispielsweise aus der WO 99/26567 bekannt. Die bekannte Vorrichtung weist ein angeschrägtes distales Ende auf, um entweder - insbesondere in Verbindung mit einer metallischen Ausführung der Vorrichtung - bei der Implantation selbst als Schneide zu wirken. Alternativ hierzu ist noch eine Implantationsmethode offenbart, bei der die Vorrichtung über eine Nadel geschoben ist und die Punktion der Sklera mit Hilfe der Nadel erfolgt. Einerseits ist ein angespitztes distales Ende der Vorrichtung ungewünscht, da hierdurch die Gefahr von Reizungen und Unverträglichkeiten der implantierten Vorrichtung vergrößert werden. Des weiteren sind die in der WO 99/26567 offenbarten Möglichkeiten zur Vergrößerung des Durchflußwiderstandes der Drainageleitung, d.h. zur Verhinderung eines übermäßigen Druckabfalls im Inneren des Auges, mit Nachteilen behaftet. Vorgeschlagen wird die Verengung des Querschnitts der Drainageleitung im distalen Endabschnitt mit Hilfe eines länglichen Reduzierkörpers, der lediglich einen bzw. mehrere kleinere Restquerschnitte freiläßt. Eine Direktpunktionstechnik ist bei einer solchen Ausgestaltung nur dann möglich, wenn das distale Ende der Vorrichtung einerseits scharf, d.h. selbstschneidend, ausgebildet ist, und andererseits, aus einem entsprechend harten Material besteht. Beide Eigenschaften sind in bezug auf die Verträglichkeit der Vorrichtung nach deren Implantation als sehr nachteilig anzusehen.

Die WO 98/30181 beschreibt des weiteren eine Kammerwasser-Drainagevorrichtung, die einen ovalförmigen oder abgerundet rautenförmigen Querschnitt besitzt. Das Drainagerohr besitzt - ausgehend von der Halteplatte - einen anwachsenden Querschnitt, der nach einem Maximum in Richtung auf das distale Ende wieder abnimmt. Mit beiden Maßnahmen soll eine Verbesserung der Abdichtung der Vorrichtung im implantierten Zustand erreicht werden. Die Implantation der Vorrichtung erfolgt dabei in der Weise, daß zunächst eine Inzision in die Sklera eingebracht wird, in die in einem zweiten Schritt das Implantat eingesetzt wird. Bei dieser Technik einer länglichen Inzision sind unerwünscht große Einschnittlängen erforderlich, um das Implantat einsetzen zu können. Außerdem ist eine Dichtheit nach Einsetzen des Implantats nicht immer gewährleistet.

Ferner ist aus US-Patentschrift 5,626,559 ein Implantat mit einer pilzkopfförmigen Halteplatte und einem Druckentlastungsventil offenbart, das in der Nähe des distalen Endes der Drainageleitung angeordnet ist. Der Flüssigkeitsaustritt aus der Halteplatte erfolgt einerseits durch vier Drainageöffnungen, die in der ansonsten geschlossenen Außenseite des Pilzkopfes angeordnet sind. Des weiteren ist die Unterseite des Pilzkopfes offen gestaltet, weshalb auch hier ein Flüssigkeitsaustritt erfolgen kann. Eine Implantation dieser bekannten Vorrichtung mit Hilfe der Direktpunktionstechnik ist nicht möglich.

Bei einer aus der EP 0168201 B 1 bekannten Vorrichtung ist der Verteilkörper in Form eines Bandes ausgeführt, das um den Äquator des Augapfels herumgeführt ist und somit eine großflächige Abgabe des Kammerwassers an das das Auge umgebende Gewebe sicherstellen soll. An der Stelle, an der die Drainageleitung in das Band übergeht, befindet sich ein Druckgradienten-Begrenzungsventil, das einen vorbestimmten Öffnungsdruck aufweist. Die Leitung und das Band sind aus Silikonkautschuk medizinischer Güte oder Hydrogel wie Hydroxyäthylenmetakrylat hergestellt.

Ein Nachteil der bekannten Vorrichtung ist darin zu sehen, daß die Implantation aufgrund der Plazierung des Bandes um den gesamten Augapfel herum sehr problematisch und auch zeitaufwendig ist.

Des weiteren ist aus der EP 0 102 747 A1 eine Druckentlastungsvorrichtung bekannt, die zwischen zwei Schlauchabschnitten ein elastisch ausdehnbares und komprimierbares Reservoir zur Aufnahme von Kammerwasser aufweist. Das Reservoir ist über ein Rückschlagventil mit dem dem Auge abgewandten Schlauchstück verbunden. Sobald ein gewisser Druckgradient über dem Ventil erreicht wird, soll das Kammerwasser in wasseraufnehmendes Gewebe am Ende des zweiten Schlauchstücks abfließen. Das komprimierbare Reservoir ist in eine Halteplatte integriert, die mit einer Vielzahl von Durchbrüchen versehen ist. Bei einer durch eine externe Kraft bewirkten Komprimierung des Reservoirs wird ein Rückfluß von in dem Reservoir befindlichem Wasser in die vordere Kammer verhindert und eine Wasserabfluß in das andere Schlauchstück erreicht.

Außerdem ist es aus der EP 0 454 838 B 1 bekannt, einen keilförmig angeschrägten Drainagekörper so zu implantieren, daß er mit seiner schrägen Stirnseite nur unwesentlich in die Vorderkammer des Auges hineinragt. Das Material des Körpers weist eine solche Porigkeit auf, daß zwar eine Durchlässigkeit für unter Druck stehendes Kammerwasser gegeben ist, ohne daß es jedoch dabei zu einem Zusammensacken der vorderen Kammer kommt. Ein Nachteil dieser vorbekannten Drainage besteht darin, daß einerseits die Wahl der korrekten Porigkeit sehr schwierig ist und andererseits die Gefahr besteht, daß die Poren des Drainagekörpers im Laufe der Zeit durch sich anlagernde Körperzellen verschlossen werden, so daß es wiederum zu einer Erhöhung des Augeninnendrucks kommen kann.

Ferner zählt zum Stand der Technik noch das Glaukomimplantat, das in der EP 0 532 654 B1 beschrieben wird. Dieses Implantat weist einen plattenförmigen Körper am Ende einer Drainageleitung auf, der mit einer Sklera im Bereich des Auges vernäht wird. Der plattenförmige Körper ist aus einem elastomeren Material hergestellt und nimmt nach einer Implantation in einem gefalteten Zustand (wegen der kleineren Inzisionsgröße) aufgrund der Körperwärme am Implantationsort wieder seine ursprüngliche Plattenform an. Beide Seiten des plattenförmigen Körpers sollen mit der angrenzenden Sklera in einem möglichst guten Kontakt stehen, um eine möglichst gute Verteilung und Aufnahme des Kammerwassers zu gewährleisten. Sowohl die Drainageleitung als auch der plattenförmige Körper sind aus einem Silikonelastomer hergestellt. Als nachteilig ist es bei diesem Implantat anzusehen, daß eine Fixierung der Drainageleitung nur dadurch erreicht werden kann, daß ein mit dieser verbundenes Fixierplättchen mit dem umgebenden Gewebe vernäht wird. Des weiteren ist der Durchmesser der Drainageleitung gering, weshalb die Gefahr besteht, daß der Kammerwasserabfluß durch sich innerhalb des Lumens der Leitung ablagernde Körperzellen verhindert wird.

Der Erfindung liegt die Aufgabe zugrunde, eine Kammerwasser-Drainagevorrichtung vorzuschlagen, die sich einfach mit Hilfe der sog. "Over-the-needle-Technik" implantieren läßt, eine sichere Verankerung in der implantierten Position gewährleistet und bei der die Gefahr einer Verstopfung durch Körperzellen auch nach längerer Zeit sehr gering ist.

Ausgehend von einer Vorrichtung der eingangs beschriebenen Art wird diese Aufgabe erfindungsgemäß dadurch gelöst, daß die Drainageleitung mit einem Druckentlastungsventil versehen ist, das einen Kammerwasserabfluß erst oberhalb eines bestimmten Drucks erlaubt, während der Implantation der Vorrichtung von einer Nadel zerstörungsfrei durchdringbar und dabei reversibel in seinen Öffnungsquerschnitt im wesentlichen auf den Querschnitt der Drainageleitung aufweitbar ist.

Die Vorrichtung gemäß der Erfindung läßt sich trotz der Halteplatte aufgrund deren hinreichend großer Durchtrittsöffnung auf dem Wege der Direktpunktionstechnik auf sehr einfache Weise, beispielsweise mit Hilfe einer Trokarnadel, implantieren. Dabei wird die erfindungsgemäße Vorrichtung im Gegensatz zu den im Durchmesser kleinen Drainageleitungen, die nach dem Stand der Technik im Wege einer "Through-the-needle-Technik" eingeführt werden, auf dem Wege der sogenannten "Over-the-needle-Technik" implantiert. Dies ermöglicht zum einen einen größeren Durchmesser der Drainageleitung sowie zum anderen die Existenz radial über deren Außendurchmesser vorstehender Elemente wie z. B. die zur Verankerung und Abstützung der Vorrichtung parallel zur Sklera vorgesehene Halteplatte. Aufgrund dieser Halteplatte ist ein Vernähen der Kammerwasser-Drainagevorrichtung gemäß der Erfindung nicht erforderlich. Der vergleichsweise große Innendurchmesser der Drainageleitung, der durch die "Over-the-needle-Technik" ermöglicht wird, reduziert die Gefahr, daß der freie Querschnitt der Drainageleitung im Laufe der Zeit durch Anlagerung von Körperzellen zu sehr reduziert wird.

Da die Halteplatte proximalen Abschluß der Vorrichtung und zugleich den Verteilkörper bildet, ist der Anschluß einer weiteren Drainageleitung an die Halteplatte nicht erforderlich. Die Halteplatte befindet sich im implantierten Zustand der Vorrichtung zwischen der Sklera und der Konjunktiva und sorgt infolge eines möglichst großflächigen Kontakts mit den vorgenannten Gewebeschichten für eine sichere Ableitung des aus der Durchtrittsöffnung austretenden Kammerwassers.

Die besondere Ausgestaltung des Druckentlastungsventils in der Art, daß dieses während der Implantation der Vorrichtung mit Hilfe der sogenannten "Over-the-needle-Technik" zerstörungsfrei dilatiert wird, gestattet einen vergleichsweise großen Querschnitt der Drainageleitung und verhindert gleichzeitig, daß der Augeninnendruck aufgrund eines ungehinderten Kammerwasserabflusses zu stark abfällt. Bewegliche Teile des Druckentlastungsventils werden beim Durchführen der Nadel radial nach außen verlagert, um einen hinreichend großen zentralen Durchtrittsquerschnitt für die Nadel zu schaffen. Bei Verwendung elastischer Kunststoffmaterialien läßt sich der Außendurchmesser der Nadel nahezu ebenso groß wählen wie der Innendurchmesser der Drainageleitung, da die radial nach außen verdrängten Teile des Druckentlastungsventils im Bereich ihrer Anlage an den Innenmantel der Drainageleitung für eine geringfügige Aufweitung der Drainageleitung sorgen. In einem an das Druckentlastungsventil angrenzenden Bereich, der dem distalen Ende der Vorrichtung zugewandt ist, kann der Drainagekanal mit einer Querschnittsvergrößerung versehen sein, um die ausgelenkten Teile des Druckentlastungsventils aufnehmen zu können, ohne daß hierbei ein zu großer Druck ausgeübt werden muß. Alternativ hierzu ist es aber auch möglich, daß die Drainageleitung einen über ihre gesamte Länge - abgesehen von dem Bereich des Druckentlastungsventils selbst - im wesentlichen konstanten Querschnitt besitzt. Bei hinreichender Elastizität der Wandung und der Ventilbauteile können letztere die Wandung beim Einschieben der Implantationsnadel nach außen verdrängen, d.h. aufweiten.

Gemäß einer Ausgestaltung der Erfindung ist vorgesehen, daß die Halteplatte auf ihrer der Drainageleitung abgewandten Seite eine Mikrostruktur aufweist. Hierdurch wird eine sichere Verteilung des Kammerwassers über die betreffende Oberfläche der Halteplatte gewährleistet.

Vorzugsweise besteht die Mikrostruktur darin, daß die Halteplatte mit strahlenförmig von der Durchtrittsöffnung aus nach außen verlaufenden Rillen versehen ist. Die Tiefe der Rillen kann in Richtung des Außenrandes der Halteplatte abnehmen, während die Breite der Rillen in dieselbe Richtung vorzugsweise zunimmt.

Neben einer scheibenförmigen Gestalt der Halteplatte ist eine bevorzugte Ausführungsform darin zu sehen, daß die Halteplatte im Querschnitt pilzkopfförmig ist.

Die Gefahr einer Verstopfung der Drainageleitung ist besonders gering, wenn ihr Innendurchmesser etwa 0,5 bis 2 mm beträgt. Die Wandstärke sollte etwa zwischen 0,05 und 0,2 mm betragen. Eine sichere Ableitung des Kammerwassers wird erzielt, wenn der Außendurchmesser der Halteplatte etwa 1 bis 5 mm beträgt.

Eine besonders gute Anpassung der Kammerwasser-Drainagevorrichtung an die Anatomie des Auges liegt dann vor, wenn die Drainageleitung einen Knick in ihrem Verlauf aufweist, wobei der Knickwinkel zwischen 10° und 20° vorzugsweise ca. 15° beträgt. Auf diese Weise kann ein Kontakt des distalen Endes der Drainageleitung mit der leicht irritierbaren Iris auch dann vermieden werden, wenn die Vorrichtung in einem Randbereich der Vorderkammer implantiert wird. Aufgrund der Elastizität des verwendeten Kunststoffmaterials wird die Vorrichtung während des Implantationsvorgangs auf der Nadel in eine gestreckte Stellung überführt und nimmt anschließend wieder die abgeknickte Position ein.

Die Erfindung weiter ausgestaltend ist vorgesehen, daß die Drainageleitung an ihrem distalen Ende mit radial ausgerichteten Drainageöffnungen in ihrem Mantel versehen ist.

Gemäß einer bevorzugten Ausführungsform der erfindungsgemäßen Vorrichtung ist diese aus einem biokompatiblen Polyurethan oder Resign aus Polyurethan und Silikon oder Polyurethan und Polycarbonat hergestellt. Polyurethan kann beispielsweise durch Zusatz von Bariumsulfat röntgenkontrastfähig ausgeführt sein. Des weiteren weist Polyurethan ein thermosensibles Verhalten auf, wodurch eine für die Implantation notwendige Rigidität der Drainage gewährleistet wird. Nach Erwärmung auf die Körpertemperatur wird das Material weich und verhindert dadurch Reizungen der Intima im Auge. Außerdem besitzt Polyurethan besondere Struktureigenschaften, die es erlauben, die Oberfläche der Drainage so einzustellen und zu dotieren, daß keine oder nur in geringem Umfang Zellen auf den Oberflächen anhaften. Eine Verlegung bzw. Verstopfung des freien Querschnitts, die zu einem erneuten Druckanstieg in der Vorderkammer des Auges führen könnte, wird somit sicher verhindert.

Vorzugsweise wird für die erfindungsgemäße Vorrichtung ein thermosensibles Polyurethan verwendet, das bei Raumtemperatur eine Shore-Härte von kleiner als 65 D und bei 37°C eine Shore-Härte von kleiner als 70A besitzt.

Die Erfindung weiter ausgestaltend ist vorgesehen, daß die Drainageleitung in Längsrichtung betrachtet an ihrem äußeren Mantel mit sich radial nach außen erstreckenden Erhebungen versehen ist. Diese Erhebungen bewirken eine Verankerung der Drainageleitung innerhalb der Sklera und verhindern somit ein Herauswandern der Vorrichtung aus dem Auge. Aufgrund der Halteplatte wird hingegen sichergestellt, daß die Drainageleitung nicht zu weit in die Vorderkammer eindringen kann. Vorzugsweise handelt es sich bei den Ausformungen um abgerundete, umlaufende Wülste, die durch zylindrische Abschnitte voneinander getrennt sind.

Bei einer bevorzugten Ausführungsform ist die Halteplatte ungefähr senkrecht zu dem proximalen Endabschnitt der Drainageleitung ausgerichtet. Eine derartige Ausrichtung ist insbesondere dann sinnvoll, wenn die Drainageleitung einen Knick aufweist und daher auch nach einem zunächst zu der Halteplatte senkrechten Abschnitt in einen geneigt hierzu verlaufenden Abschnitt übergehen kann.

Wenn das Druckentlastungsventil in der Nähe des proximalen Endes der Drainageleitung angeordnet ist, lassen sich eventuelle zelluläre Anlagerungen nach einer gewissen Verweilzeit des Implantats im Körper von außen, beispielsweise mit Hilfe eines geeigneten Lasers, wieder davon entfernen. Eine Explantation der Vorrichtung bzw. die Implantation einer neuen Vorrichtung ist in diesem Falle überflüssig.

Um die Implantation der erfindungsgemäßen Vorrichtung mit Hilfe einer Trokarnadel zu erleichtern, sollte das distale Ende der Drainageleitung konisch ausgeformt sein. Wird ein mit Hilfe eines - vorzugsweise distal abgerundeten - Mandrins verschlossener Trokar verwendet, so kann beim Durchstoßen der Sklera ein unkontrollierter Kammerwasserabfluß verhindert werden. Durch kontrolliertes Zurückziehen des Mandrins kann jedoch ein Kammerwasserabfluß und somit eine hinreichend tiefe Punktion der Sklera festgestellt werden. Wird ein mit Hilfe eines sog. Blutrückflußventils ausgestatteter Trokar verwendet, so kann beim Durchstoßen der Sklera durch Liquoraustritt in die Ventilkammer die korrekte Plazierung kontrolliert werden.

Ferner ist gemäß der Erfindung noch vorgesehen, daß die Oberfläche der Vorrichtung auf elektrophysikalische und/oder chemische Weise oberflächenbehandelt und/ oder dotiert ist. Ebenso läßt sich die gesamte Kunststoffmatrix vollständig mit Wirkstoffen dotieren, die über eine definierte Zeit abgegeben werden. Auf diese Weise lassen sich eine Vielzahl verschiedener Eigenschaften der Vorrichtung einstellen.

Schließlich wird nach der Erfindung noch vorgeschlagen, daß das Druckentlastungsventil ein mehrfächriges Lippenventil ist, dessen Lippensegmente jeweils in einem Öffnungsbereich, der der Mittelachse der Drainageleitung zugewandt ist, aus einem härteren Kunststoffmaterial und in einem an die Wandung der Drainageleitung angrenzenden Stützbereich aus einem weicheren Kunststoffmaterial bestehen. Hierdurch wird eine ausreichende Elastizität der Segmente erreicht und dennoch eine sichere Anlage der Dichtlippen im Bereich mit dem steiferen Kunststoffmaterial, wo die Wandstärke der Lippen in der Regel reduziert ist.

Die Erfindung wird nachfolgend anhand eines Ausführungsbeispiels einer Kammerwasser-Drainagevorrichtung, die in der Zeichnung dargestellt ist, näher erläutert. Es zeigt:
- Fig. 1: einen Querschnitt durch ein menschliches Auge mit einer Kammerwasser-Drainagevorrichtung im implantierten Zustand;
- Fig. 2: eine Seitenansicht der Vorrichtung gemäß Fig. 1;
- Fig. 3: einen Querschnitt durch eine Halteplatte der Vorrichtung gemäß Fig. 2;
- Fig. 4: eine Draufsicht auf die Halteplatte der Vorrichtung gemäß Fig. 2;
- Fig. 5: einen Längsschnitt durch eine Drainageleitung mit einem Deckelventil;
- Fig. 6: wie Fig. 5, jedoch mit einem Lippenventil
- Fig. 6a: eine Draufsicht auf das Lippenventil gemäß Fig. 6 und
- Fig. 7a und Fig. 7b: jeweils einen Längsschnitt durch eine Trokarnadel mit Stumpfschliff und innenliegendem distal abgerundetem Mandrin sowie die zur Implantation aufgeschobene Vorrichtung mit geschlossenem bzw. offenem Druckentlastungsventil.

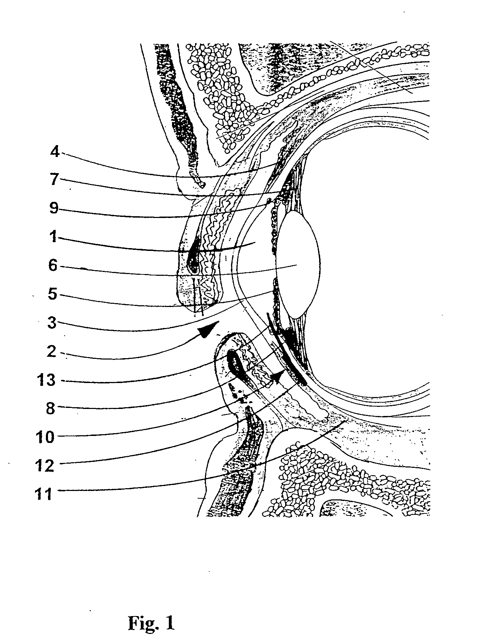

Wie aus Figur 1 ersichtlich ist, wird die vordere Kammer 1 eines Auges 2 nach vorne von der durchsichtigen Hornhaut 3 und der darüber angeordneten Konjunktiva (Bindehaut) 4, und zum Augapfel hin von der Iris 5 und der Linse 6 begrenzt, die mittels Zonulafasern 7 am Ziliarkörper 8 befestigt ist. Das Kammerwasser in der vorderen Kammer 1 und der damit in Verbindung stehenden hinteren Kammer wird ständig erneuert, wobei ein Abfluß des überschüssigen Kammerwassers beim gesunden Auge durch den sogenannten Schlemmkanal 9 erfolgt, so daß sich ein konstanter Innendruck in der vorderen Kammer 1 einstellt.

In dem in Figur 1 dargestellten Auge befindet sich eine Kammerwasser-Drainagevorrichtung 10 in dem Bereich des Auges, in dem die durchsichtige Hornhaut 3 in die undurchsichtige Sklera (Lederhaut) 11 übergeht. Die Vorrichtung 10 ist im implantierten Zustand so ausgerichtet, daß ihre Halteplatte 12 im Grenzbereich zwischen der Sklera 11 und der Bindehaut 4, und zwar im wesentlichen parallel zu den beiden vorgenannten Schichten, angeordnet ist. Eine Drainageleitung 13 durchdringt mit einem ersten proximalen Abschnitt die Sklera 11 und knickt dann in einem zweiten distalen Abschnitt ab, um ungefähr parallel zur Iris 5 zu verlaufen. Eine Berührung mit der leicht irritierbaren Iris 5 wird auf diese Weise vermieden.

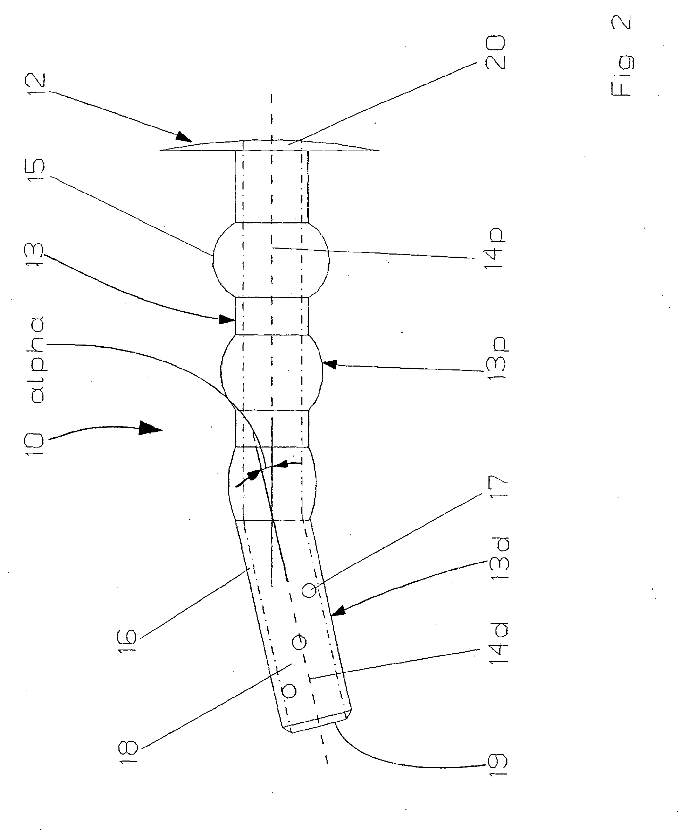

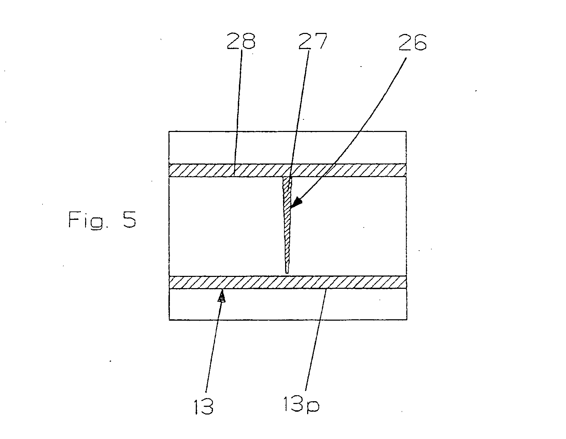

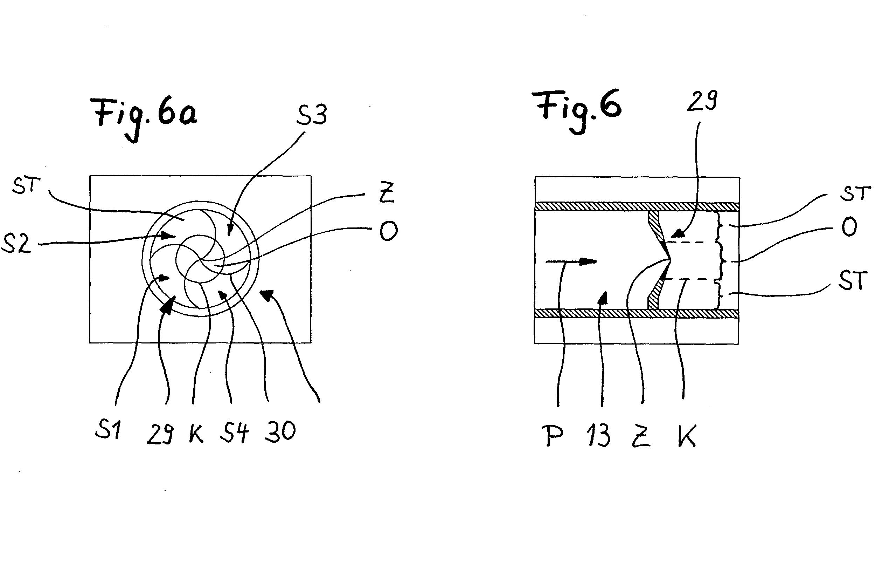

Figur 2 läßt sich entnehmen, daß die Halteplatte 12 am proximalen Ende der Vorrichtung 10 im Querschnitt pilzkopfförmig ist. Die Drainageleitung 13 weist einen distalen Abschnitt 13d und einen proximalen Abschnitt 13p auf, deren Längsachsen 14d und 14p sich in einem Winkel α von 15° schneiden. Der proximale Abschnitt 13p der Drainageleitung 13 befindet sich im wesentlichen innerhalb der Sklera 11 und ist zur besseren Verankerung im Skleragewebe mit drei wulstförmigen umlaufenden Ausformungen 15 versehen, die eine Axialverschiebung unterbinden. Innerhalb des proximalen Abschnitts 13p, und zwar im Bereich des ersten zylindrischen Abschnitts vor der - vom proximalen Ende aus gesehen ersten Ausformung 15 -, befindet sich ein in Fig. 2 nicht dargestelltes Druckentlastungsventil, dessen Aufbau in den Figuren 5 und 6 gezeigt ist. Der äußere Mantel 16 ist im Bereich des distalen Abschnitts 13d mit einer Mehrzahl von radial ausgerichteten Drainageöffnungen 17 versehen. Diese Drainageöffnungen 17 kommunizieren mit dem inneren Lumen 18 der Drainageleitung 13, die an der distalen Stirnseite 19 offen ist. Am proximalen Ende der Vorrichtung 10 geht das Lumen 18 ohne Sprünge in eine koaxial zu dem Lumen 18 ausgerichtete Durchtrittsöffnung 20 über, die dieselbe Querschnittsfläche wie das übrige Lumen 18 besitzt.

Die Vorrichtung 10 ist insgesamt einstückig ausgebildet und aus einem biokompatiblen thermosensiblen Polyurethan hergestellt, das bei Raumtemperatur eine Shore-Härte von ca. 65D und bei einer Temperatur von 37° C eine Shore-Härte von ca. 60A besitzt. Bei Raumtemperatur ist die Vorrichtung 10 somit hinreichend steif, um mit Hilfe einer Punktionstechnik implantierbar zu sein, wohingegen nach einer Erwärmung auf die Körpertemperatur die Vorrichtung 10 weich und elastisch genug ist, um Reizungen des Auges 2 zu vermeiden. Die Oberfläche der Vorrichtung 10 ist auf elektrophysikalischem oder chemischem Wege behandelt, um die Anhaftrate von Körperzellen zu reduzieren und somit ein Verstopfen des inneren Lumens 18 der Drainageleitung 13 zu verhindern.

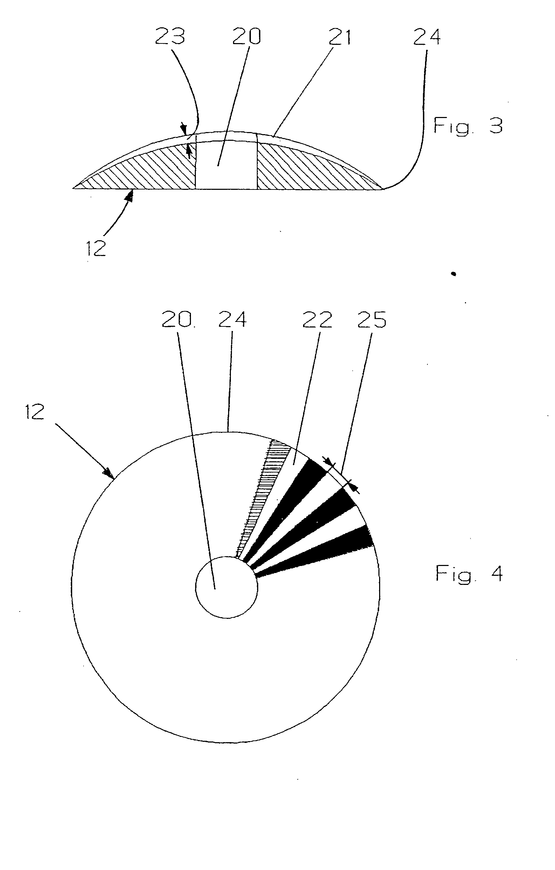

Die Figuren 3 und 4 verdeutlichen, daß die pilzkopfförmige Halteplatte 12 an ihrer der Drainageleitung 13 abgewandten Oberfläche 21 mit einer Oberflächenstruktur versehen ist, die aus strahlenförmig um die Durchtrittsöffnung 20 angeordneten im Querschnitt rechteckförmigen Rillen 22 versehen ist. Während die Höhe 23 der Rillen 22 von der Durchtrittsöffnung 20 zu einem umlaufenden Rand 24 hin abnimmt, nimmt die Breite 25 von der Durchtrittsöffnung 20 zum Rand 24 hin zu. Insgesamt bleibt die Querschnittsfläche daher ungefähr konstant, wohingegen die Kontaktfläche mit dem umgebenden Bindehautgewebe sich nach außen hin vergrößert.

Die Figuren 5 und 6 zeigen zwei unterschiedliche Ausführungsformen jeweils eines Druckentlastungsventils, das im Bereich des proximalen Abschnitts 13p der Drainageleitung 13 angeordnet ist. Während das Ventil gemäß Figur 5 als Deckelventil 26 ausgebildet ist, das über einen vergleichsweise kleinen Kontaktwinkelbereich 27 mit der Wandung 28 der Drainageleitung 13 verbunden ist, ist das Ventil gemäß Figur 6 in Form eines Lippenventils 29 ausgebildet, dessen Lippen entlang von wellenförmigen Dichtspalten 30 dichtend aneinanderstoßen und im Falle eines hinreichenden Überdrucks zunächst zentrumsnahe Durchtrittsspalte freigeben. Der Verschluß der Dichtspalte 30 erfolgt mit Hilfe der elastischen Vorspannung der in ihrer Wandstärke in Richtung auf die Mittelachse der Drainageleitung 13 abnehmenden Lippen.

Wie sich insbesondere aus Figur 6a ergibt, besteht das Lippenventil 29 aus vier Lippensegmenten S1 bis S4, die sich in ihren einzelnen Winkelbereichen auf insgesamt 360° ergänzen. Die Dichtspalte 30 verlaufen jeweils sichelförmig und ergänzen sich paarweise zu einer S-förmigen bzw. wellenförmigen Gestalt. Wie in Figur 6a durch den Kreis K und in Figur 6 durch die geschwärzten Spitzenden der Lippensegmente S1 bis S4 angedeutet ist, bestehen die Lippensegmente S1 bis S4 jeweils in einem Öffnungsbereich O aus einem härterem und in einem kreisringförmigen Stützbereich ST aus einem im Vergleich hierzu weicheren Kunststoffmaterial. Hierdurch wird auch im Bereich der im Öffnungsbereich O vorherrschenden dünnen Wandstärken eine hineichende Stabilität der Dichtlippen und eine zuverlässige Abdichtung gewährleistet.

Übersteigt der Druck in dem dem distalen Ende der Vorrichtung zugewandten Abschnitt der Drainageleitung 13, so öffnet sich das Lippenventil 29 zunächst im Bereich des Zentrums Z, wo die Wandstärke der Lippen am geringsten ist. Mit zunehmenden Druck kann auch eine weitere Öffnung des Lippenventils 29 eintreten, das in diesem Fall einen kreuzförmigen Öffnungsquerschnitt entsprechend der Dichtspalte 30 aufweist. Das Kammerwasser kann bei geöffnetem Lippenventil 29 in Richtung des Pfeils P abfließen.

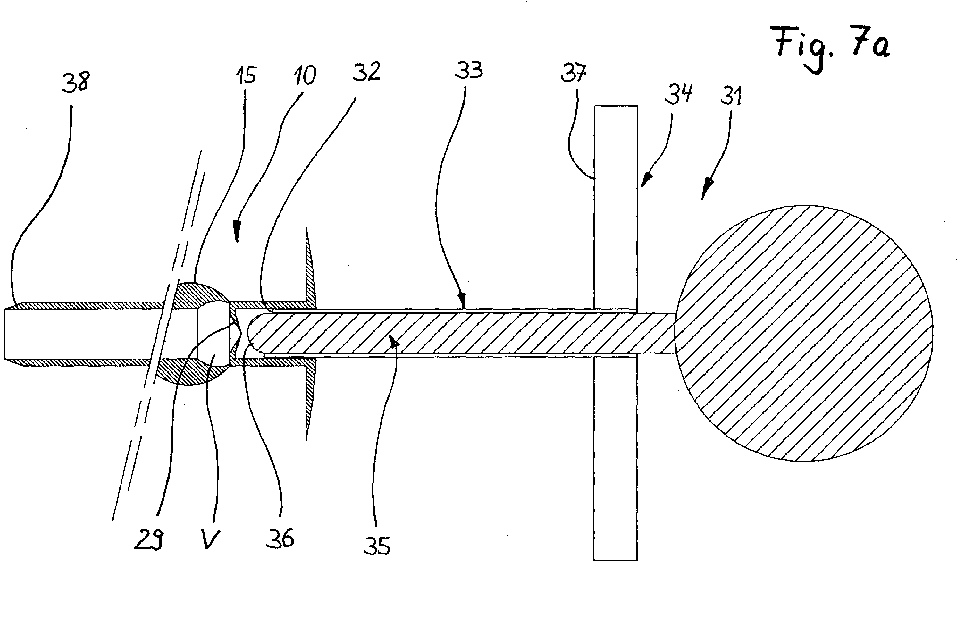

Figur 7a zeigt eine Trokarvorrichtung 31 im Längsschnitt, die zur Implantation der Vorrichtung 10 im Wege der Direktpunktionstechnik dient. Die Trokarvorrichtung 31 besteht aus einer hohl ausgeführten und an ihrem distalen Ende 32 stumpf angeschliffenen Trokarnadel 33, die an ihrem proximalen Ende mit einem radial nach außen vorstehenden Kragen 34 versehen ist. Der Schliff der Trokarnadel 33 weist vorzugsweise die Form einer Crawfordnadel auf, die u.a. zur gewebeschonenden Punktion der Duramater bei Epiduralanästhesie verwendet wird.

Innerhalb der Trokarnadel 33 befindet sich ein distal abgerundeter Mandrin 35, dessen Außendurchmesser geringfügig kleiner als der Innendurchmesser der Trokarnadel 33 ist.

Der Außendurchmesser der Trokarnadel 33 ist so bemessen, daß er ungefähr dem Innendurchmesser des Lumens 18 der Vorrichtung 10 entspricht. Die Länge der Trokarnadel 33-gemessen zwischen der distalen Stirnfläche 37 des Kragens und dem nächstgelegenen Punkt der schräg verlaufenden distalen Stirnfläche - entspricht der gestreckten Länge der Drainageleitung 13. Das distale Ende 38 der Drainageleitung 13 ist konisch angespitzt um das Eindringen in das Gewebe bei der Punktion zu erleichtern.

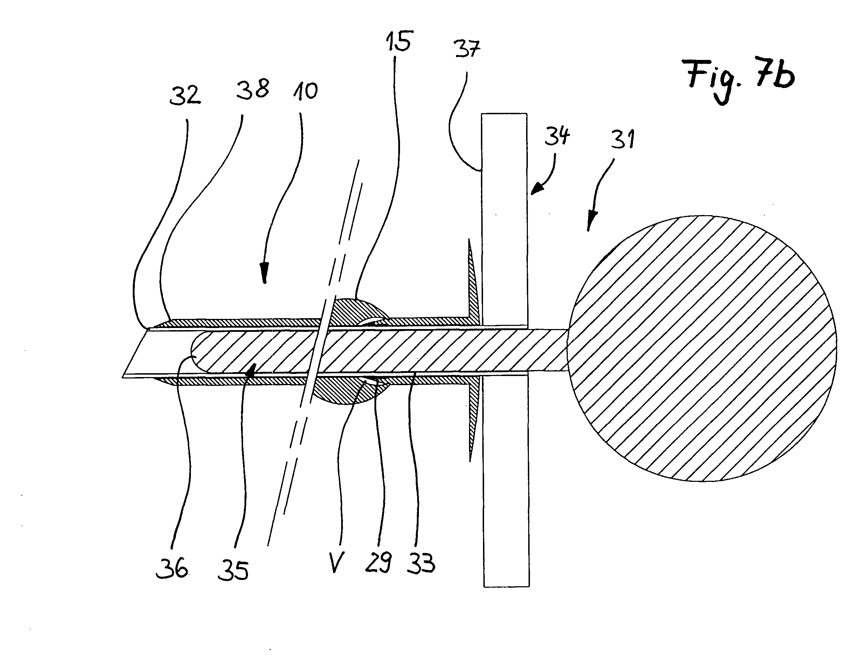

Beim Aufschieben der Vorrichtung 1 auf die Trokarnadel 33 befindet sich der Mandrin 35 in einer leicht vorgeschobenen Stellung. Aufgrund des abgerundeten distalen Endes des Mandrins wird das Lippenventil 29, das in seiner geschlossenen Ausgangsstellung die Drainageleitung 13 vollständig verschließt, geöffnet, wobei sich die Lippensegmente S1 bis S4 in Richtung auf die Wandung W der Drainageleitung 13 verlagern. In einem Abschnitt hinter dem Lippenventil im Bereich einer wulstförmigen Ausformung 15 befindet sich eine Querschnittsvergrößerung V, die in ihrem Querschnitt so bemessen ist, daß sie die ausgelenkten Lippensegmente S1 bis S4 im weiter vorgeschobenen Zustand der Trokareinrichtung 31 aufnehmen kann. Dieser Zustand ist in Figur 7b dargestellt. Im Anschluß an den Mandrin 35 wird auch die Trokarnadel 33 vorgeschoben, so daß das Lippenventil 29 einen Öffnungsquerschnitt besitzt, der dem Querschnitt der Trokarnadel 33 entspricht.

Aufgrund der geschlossenen Ausführung von Trokarvorrichtung 31 und darüber geschobener Vorrichtung 10 wird das Gewebe bei der Punktion nur minimal verletzt. Der Mandrin 35 bewirkt einerseits einen Verschluß der Trokarnadel 33, so daß ein unabsichtlicher Abfluß von Kammerwasser nach Durchstoßen der Sklera 11 nicht zu befürchten ist. Andererseits läßt sich durch vorsichtiges Zurückziehen des Mandrins 35 überprüfen, ob die Sklera 11 bereits vollständig durchstoßen ist und sich die Spitze der Trokarvorrichtung 31 somit bereits in der vorderen Kammer 1 des Auges 2 befindet.

Aufgrund der elastischen Materialeigenschaften des Polyurethans läßt sich die gesamte Drainageleitung 13 einschließlich deren abgewinkelten distalen Abschnitts 13d in eine gestreckte Anordnung auf der Trokarnadel 33 überführen. Nach dem Zurückziehen der Trokarvorrichtung 31 nimmt die Vorrichtung 10 sodann wieder ihre abgewinkelte Form ein und kollidiert daher nicht mit der Iris 5. Das Lippenventil geht nach Zurückziehen der Trokarnadel 33 ebenfalls wieder in die in Fig. 7a gezeigte Ausgangsstellung zurück.

## Patentansprüche

1. Kammerwasser-Drainagevorrichtung (10) für ein Auge (2) mit einer die Sklera (11) durchdringenden und ein inneres Lumen (18) begrenzenden Drainageleitung (13), die so plazierbar ist, daß ihr distales Ende (19) in der vorderen oder hinteren Kammer (1) des Auges (2) angeordnet ist, und einem an das proximale Ende der Drainageleitung (13) angeschlossenen Verteilkörper, der außerhalb der vorderen oder hinteren Kammer (1) angeordnet ist und einer Verteilung des abgeleiteten Kammerwassers an ihn umgebendes Gewebe bewirkt, wobei der Verteilkörper eine Halteplatte (12) ist, die fest mit der Drainageleitung (13) verbunden ist, radial über deren Außendurchmesser vorsteht und eine Durchtrittsöffnung (20) aufweist, die koaxial zu der Drainageleitung (13) verläuft und eine Querschnittsfläche besitzt, die mindestens der minimalen Querschnittsfläche des Lumens (18) der Drainageleitung (13) entspricht, **dadurch gekennzeichnet, daß** die Drainageleitung (13) mit einem Druckentlastungsventil (26, 29) versehen ist, das einen Kammerwasserabfluß erst oberhalb eines bestimmten Drucks erlaubt, das während der Implantation der Vorrichtung (1) von einer Nadel zerstörungsfrei durchdringbar und das dabei reversibel in seinem Öffnungsquerschnitt im wesentlichen auf den Querschnitt der Drainageleitung aufweitbar ist.

2. Vorrichtung nach Anspruch 1, **dadurch gekennzeichnet, daß** die Halteplatte (12) auf ihrer der Drainageleitung (13) abgewandten Seite eine Mikrostruktur aufweist.

3. Vorrichtung nach Anspruch 2, **dadurch gekennzeichnet, daß** die Halteplatte (12) mit strahlenförmig von der Durchtrittsöffnung (20) aus nach außen verlaufenden Rillen (22) versehen ist.

4. Vorrichtung nach Anspruch 3, **dadurch gekennzeichnet, daß** die Tiefe (23) der Rillen (22) in Richtung des Außenrandes (24) der Halteplatte (12) abnimmt und die Breite (25) der Rillen (22) in dieselbe Richtung zunimmt.

5. Vorrichtung nach einem der Ansprüche 1 bis 4, **dadurch gekennzeichnet, daß** die Halteplatte (12) im Querschnitt pilzkopfförmig ist.

6. Vorrichtung nach einem der Ansprüche 1 bis 5, **dadurch gekennzeichnet, daß** der Innendurchmesser der Drainageleitung etwa 0,5 bis 2 mm, deren Wandstärke etwa zwischen 0,05 und 0,2 mm, und der Außendurchmesser der Halteplatte (12) etwa 1 bis 5 mm beträgt.

7. Vorrichtung nach einem der Ansprüche 1 bis 6, **dadurch gekennzeichnet, daß** die Drainageleitung (13) einen Knick in ihrem Verlauf aufweist, wobei der Knickwinkel α zwischen 10 und 20°, vorzugsweise 15° beträgt.

8. Vorrichtung nach einem der Ansprüche 1 bis 7, **dadurch gekennzeichnet, daß** die Drainageleitung (13) an ihrem distalen Ende (19) mit radial ausgerichteten Drainageöffnungen (17) in ihrem Mantel versehen ist.

9. Vorrichtung nach einem der Ansprüche 1 bis 8, **dadurch gekennzeichnet, daß** die Vorrichtung (10) aus biokompatiblem Polyurethan oder aus Resigns aus Polyurethan und Silikon oder Polyurethan und Polycarbonat hergestellt ist.

10. Vorrichtung nach Anspruch 9, **dadurch gekennzeichnet, daß** das Polyurethan thermosensibel ist und bei Raumtemperatur eine Shore-Härte von kleiner als 65D und bei 37°C eine Shore-Härte von kleiner als 70A besitzt.

11. Vorrichtung nach einem der Ansprüche 1 bis 10, **dadurch gekennzeichnet, daß** die Drainageleitung (13) in Längsrichtung betrachtet, an ihrem äußeren Mantel mit sich radial nach außen erstreckenden Ausformungen (15) versehen ist.

12. Vorrichtung nach Anspruch 11, **dadurch gekennzeichnet, daß** die Ausformungen (15) abgerundete, umlaufende Wülste sind.

13. Vorrichtung nach einem der Ansprüche 1 bis 12, **dadurch gekennzeichnet, daß** das distale Ende (19) der Drainageleitung (13) konisch ausgeformt ist.

14. Vorrichtung nach einem der Ansprüche 1 bis 13, **dadurch gekennzeichnet, daß** die Vorrichtung auf elektrophysikalische und/ oder chemische Weise oberflächenbehandelt und/oder dotiert ist oder die gesamte Kunststoffmatrix dotiert ist.

15. Vorrichtung nach Anspruch 1 bis 14, **dadurch gekennzeichnet, daß** die Halteplatte (12) ungefähr senkrecht zu dem proximalen Endabschnitt der Drainageleitung (13) ausgerichtet ist.

16. Vorrichtung nach einem der Ansprüche 1 bis 15, **dadurch gekennzeichnet, daß** das Druckentlastungsventil (26, 29) in der Nähe des proximalen Endes der Drainageleitung (13) angeordnet ist.

17. Vorrichtung nach Anspruch 1 bis 16, **dadurch gekennzeichnet, daß** das Druckentlastungsventil ein mehrfächriges Lippenventil (29) ist, dessen Lippensegmente (S1 bis S4) jeweils in einem Öffnungsbereich (O), der der Mittelachse der Drainageleitung (13) zugewandt ist, aus einem härteren Kunststoffmaterial und in einem an die Wandung der Drainageleitung (13) angrenzenden Stützbereich (ST) aus einem weicheren Kunststoffmaterial bestehen.
